Europäisches Patentamt

European Patent Office

Office européen des brevets

(19)

(11) Publication number: **0 394 510**
**A1**

(12) # EUROPEAN PATENT APPLICATION

(21) Application number: **89107400.7**

(22) Date of filing: **24.04.89**

(51) Int. Cl.⁵: **G01N 33/574, G01N 33/545**

(43) Date of publication of application:
**31.10.90 Bulletin 90/44**

(84) Designated Contracting States:
**AT BE CH DE ES FR GB GR IT LI NL SE**

(71) Applicant: **MOCHIDA PHARMACEUTICAL CO., LTD.**
**7, Yotsuya 1-chome**
**Shinjuku-ku Tokyo 160(JP)**

(72) Inventor: **Katamine, Tomoaki**
**Urban-aiko No. 603 39-12, Minamiurawa**
**2-chome**
**Urawa-shi Saitama(JP)**
Inventor: **Sato, Hiroshi**
**3-201, 3-1-1, Sakurada Washimiya-cho**
**Kitakatsushika-gun Saitama(JP)**
Inventor: **Mochida, Ei**
**5-4, Komagome 2-chome**
**Toshima-ku Tokyo(JP)**

(74) Representative: **Henkel, Feiler, Hänzel & Partner**
**Möhlstrasse 37**
**D-8000 München 80(DE)**

(54) **Method for assaying a tumor-associated antigen in a minute amount of nipple discharge and device for such an assay.**

(57) Concentration of the tumor-associated antigen selected from the group consisting of CA15-3, CA125, CA72-4, CA54/61, CA602, CA19-9, TPA, MCA, MSA, and ATM-1 in the nipple discharge is assayed by means of an antigen-antibody reaction using an antibody and/or a fragment thereof against said tumor-associated antigen, and said antigen-antibody reaction is effected on a sheet-form solid phase.

The assay of the concentration of the tumor-associated antigen in the nipple discharge is effected by use of a device comprising a sheet-form solid phase having at least one reaction area defined thereon for an antigen-antibody reaction between an antibody and the tumor-associated antigen in the nipple discharge.

EP 0 394 510 A1

# Method for Assaying a Tumor-Associated Antigen in a Minute Amount of Nipple Discharge and Device for Such an Assay

## BACKGROUND OF THE INVENTION

This invention relates to a method for assaying a tumor-associated antigen selected from the group consisting of CA (cancer antigen) 15-3, CA (cancer antigen) 125, CA (carbohydrate antigen) 72-4, CA (carbohydrate antigen) 54/61, CA (carbohydrate antigen) 602, CA (carbohydrate antigen) 19-9, TPA (tissue polypeptide antigen), MCA (mucinous carcinoma-associated antigen), MSA (mammary serum antigen), and ATM-1 contained in a very minute amount of nipple discharge, and a device for carrying out such an assay.

Quite numerous tumor markers, namely, tumor-associated antigens are currently reported, and their concentration in blood are also assayed. Among these markers, the most widely employed tumor-associated antigen is CEA (carcinoembryonic antigen) which was reported by Gold et al. (J. Exp. Med., 121, 439, 1965). Assay of CEA concentration in blood is found to be useful for monitoring prognosis of various surgeries and determining effects of various treatments. The concentration of such a tumor-associated antigen is assayed by quantitative reactions including radioimmunoassay (RIA) and enzyme immunoassay (EIA). It is, however, very difficult to detect a cancer at an early stage by measuring the concentration of the tumor-associated antigen including CEA in blood. The known tumor-associated antigens do not exhibit high specificity for tumors, and therefore, it has been difficult to accurately diagnose a cancer by assaying the concentration of a single tumor-associated antigen in blood.

Breast cancer is one of the most common cancers, and gradually increasing. Detection of primary breast cancer at an early stage by assaying the concentration of tumor-associated antigen in blood has been very difficult. For example, CA15-3, a tumor-associated antigen of breast cancer, exhibits positive rate for early stage breast cancer of as low as 4 to 8%, as disclosed in the brochure titled "Centocore CA15-3 RIA kit" provided by TORAY-FUJI BIONICS, INC. Consequently, initial diagnosis is often delayed and the cancer is proceeded too far and metastasized to such sites as lymph node and the like, inevitably requiring treatments such as mastectomy.

Inaji et al. (Igaku-no-Ayumi, 134, 575, 1985) reports that breast cancer can be detected at an early stage by measuring CEA which is a tumor-associated antigen found in nipple discharge. In this process, the assay of CEA in nipple discharge is carried out by enzyme immunoassay (Elmotech-CEA, Mochida Pharmaceutical Co., Ltd.). This CEA assay, however, requires various equipments including reaction equipments and a spectrophotometer for determining the results of the reaction, and therefore, such a CEA assay is far from a convenient process which can be readily carried out at every facility. Further, since a calibration curve must be depicted at every examination regardless of the number of the specimens, this process has been inefficient for such a symptom as abnormal secretion from nipple wherein the specimen is less frequently generated. Moreover, an amount of at least 50 microliters of specimen is required even for Elmotech-CEA, which is an EIA kit employed for measuring CEA presently requiring a relatively small amount of specimen. The amount of nipple discharge secreted is usually as small as 10 microliters or less. The nature of the nipple discharge, particularly the viscosity, is also widely varied among specimens compared to serum specimens, and the collection of the specimen is difficult in some cases. The amount of the specimen collected has often been too small to carry out an assay, and a development of an assay which can be carried out with a minute amount of specimen has been strongly demanded.

In consideration of such a background, there is a marked social demand for development of a highly specific and potent diagnosis of the breast cancer. Particularly desired is an assay for a tumor-associated antigen which can be utilized in a mass-screening for breast cancer at group examination or dispensary of mammary gland. The assay of the tumor-associated antigen in nipple discharge would have a significant importance in such a field, and a convenient procedure for accurately measuring the tumor-associated antigen in nipple discharge which can be carried out by anyone would certainly have a clinical significance.

The inventors of the present invention have made an intensive study to overcome the above-described problems in the diagnosis of breast cancer and to develop a method for measuring tumor-associated antigens including CA15-3, CA125, CA72-4, CA54/61, CA602, CA19-9, TPA, MCA, MSA, and ATM-1 contained in nipple discharge which can fulfill the social demands that the assay can be carried out by using a minute amount of specimen at group examination or mass screening by simple operation, and found that the above-mentioned problems may be overcome by utilizing a sheet-form material as a support for the insolubilized antibody in conventional EIA so that the reaction between the antigen to be assayed may react with the antibody on the sheet-form material. The present invention is achieved on such a finding.

## SUMMARY OF THE INVENTION

An object of the present invention is to provide a method for conveniently detecting or assaying a tumor-associated antigen selected from the group consisting of CA15-3, CA125, CA72-4, CA54/61, CA602, CA19-9, TPA, MCA, MSA, and ATM-1 contained in a biological discharge, particularly in a minute amount of nipple discharge, by an immunoassay using an antigen-antibody reaction as well as a device required to carry out such a method. Further objects of the present invention is to enable the detection of breast cancer at an early stage and to fulfill the above-mentioned social demand.

According to a first aspect of the present invention, there is provided a method for assaying concentration of a tumor-associated antigen selected from the group consisting of CA (cancer antigen) 15-3, CA (cancer antigen) 125, CA (carbohydrate antigen) 72-4, CA (carbohydrate antigen) 54/61, CA (carbohydrate antigen) 602, CA (carbohydrate antigen) 19-9, TPA (tissue polypeptide antigen), MCA (mucinous carcinoma-associated antigen), MSA (mammary serum antigen), and ATM-1 contained in a minute amount of nipple discharge wherein the concentration of said tumor-associated antigen in the nipple discharge is assayed by means of an antigen-antibody reaction using an antibody or a fragment thereof against said tumor-associated antigen, and said antigen-antibody reaction is effected on a sheet-form solid phase.

According to a second aspect of the present invention, there is provided a device for assaying a tumor-associated antigen selected from the group consisting of CA15-3, CA125, CA72-4, CA54/61, CA602, CA19-9, TPA, MCA, MSA, AND ATM-1 in a minute amount of nipple discharge comprising a sheet-form solid phase having at least one reaction area defined thereon for an antigen-antibody reaction between the tumor-associated antigen in the nipple discharge and an antibody or a fragment thereof against said tumor-associated antigen.

The sheet-form solid phase may preferably be a substantially liquid impermeable plastic sheet, said plastic being selected from the group consisting of polyester, polypropylene, and a polyethylene.

## BRIEF DESCRIPTION OF THE DRAWINGS

FIG. 1a is a plan view, and FIG. 1b is a cross-sectional view of one embodiment of the device according to the present invention.

FIG. 2a is a plan view, and FIG. 2b is a cross-sectional view of another embodiment of the device according to the present invention.

FIG. 3a is a plan view, and FIG. 3b is a cross-sectional view of further embodiment of the device according to the present invention.

FIG. 4a is a plan view, and FIG. 4b is a cross-sectional view of a still further embodiment of the device according to the present invention.

FIGS. 5 through 11 are diagrams illustrating the concentration of CA15-3, CA125, CA72-4, CA54-61, CA602, CA19-9, and TPA contained in the nipple discharge collected from the patients suffering from various diseases examined in Example 1, respectively.

## DETAILED DESCRIPTION OF THE INVENTION

The method for assaying a tumor-associated antigen selected from the group consisting of CA15-3, CA125, CA72-4, CA54/61, CA602, CA19-9, TPA, MCA, MSA and ATM-1 in a minute amount of nipple discharge according to the present invention is characterized in that the concentration of said tumor-associated antigen is assayed on a sheet-form solid phase. It is the employment of the sheet-form solid phase that has enabled a minute amount of the nipple discharge to be assayed for the tumor-associated antigen. The term "minute amount" used herein designates an amount in the order of from 0.2 to 50 microliters. The nipple discharge collected from patients may be used as it is, although it may also be diluted by a factor of about 2 to 100.

3

Preferably, the method according to the present invention is carried out by a sandwich method typically comprising the steps of insolubilizing an antibody or a fragment thereof (the antibody and the fragment thereof are hereinafter generically referred to as antibody) against the tumor-associated antigen in the nipple discharge to be detected or assayed onto a substantially liquid impermeable sheet-form solid phase; applying the nipple discharge onto said antibody-insolubilized sheet-form solid phase to allow for a reaction between the insolubilized antibody and the tumor-associated antigen; applying a labelling substance comprising the antibody against said tumor-associated antigen and a labelling agent bound to the antibody onto said solid phase to allow for a reaction between the antibody contained in the labelling substance and the tumor-associated antigen, said labelling agent being identifiable by appropriate means; and thereafter removing the nipple discharge which failed to bind with the antibody insolubilized onto said solid phase and the labelling substance which failed to bind with the tumor-associated antigen by rinsing the solid sheet; and measuring the concentration of the tumor-associated antigen on the bases of the amount of the labelling substance bound to said sheet-form solid phase. The present method, however, is not limited to such a sandwich method.

An exemplary method which is not the sandwich method employs a sheet-form solid phase without any antibody insolubilized thereon. In such a method, the collected specimen is applied onto a predetermined area on the solid phase having no antibody insolubilized thereon; a labelling substance comprising the antibody having an identifiable labelling agent bound thereto is applied onto the solid phase to allow for a reaction between the antigen contained in the sample and the antibody contained in the labelling substance; the solid phase is then rinsed to remove the specimen components and the labelling substance which failed to bind with the solid phase; and the amount of the tumor-associated antigen is assayed on the bases of the labelling substance bound onto said predetermined area of the solid phase.

In the sandwich method, the antibody is less likely to be influenced by the components in the nipple discharge other than said tumor-associated antigen since the tumor-associated antigen contained in the nipple discharge immunochemically binds onto the area on the solid phase wherein the the antibody has been insolubilized. Further, the sandwich method is more sensitive than the method utilizing the solid phase with no antibody insolubilized thereto. Also the sensitivity and the concentration range measured may more readily be adjusted in the sandwich method. The sandwich method wherein the antibody is insolubilized onto the solid phase is thus desirable.

Three variations of the sandwich method can be carried out in accordance with the present invention corresponding to the reaction order of three factors, that is, the antibody insolubilized onto the sheet-form solid phase, the specimen, and the labelling substance.

The first method comprises the steps of insolubilizing the antibody against the tumor-associated antigen in the nipple discharge to be detected or assayed onto the sheet-form solid phase; applying the nipple discharge specimen onto the sheet-form solid phase having the antibody bound thereto to allow for a reaction between the specimen and the insolubilized antibody; applying the labelling substance comprising the antibody against said tumor-associated antigen and the labelling agent identifiable by appropriate means onto said solid phase to allow for a reaction between the tumor-associated antigen and the antibody contained in the labelling substance; rinsing said solid phase to remove the nipple discharge components and the labelling substance which failed to bind; and measuring the concentration of the tumor-associated antigen on the bases of the amount of the labelling substance bound to the sheet-form solid phase.

The second method comprises the steps of preliminarily applying the labelling substance comprising the antibody having the identifiable agent bound thereto onto the sheet-form solid phase having the antibody insolubilized thereon; and applying the nipple discharge specimen on the predetermined area of the thus pretreated solid phase to simultaneously effect the reaction between the specimen components and the insolubilized antibody and the reaction between the specimen components and the labelling substance. In this method, an optional step of lyophilization carried out after the application of the labelling substance onto the solid phase having the antibody insolubilized thereon may increase stability of the reagents including the labelling substance.

The third method which may be carried out in the present invention comprises the steps of mixing the collected nipple discharge specimen and the labelling substance comprising the antibody having the identifiable labelling agent bound thereto, and applying said mixture on the predetermined reaction area of the solid phase having the antibody insolubilized thereon to allow for a reaction between the tumor-associated antigen contained in said mixture and the antibody insolubilized onto the solid phase.

The substances which is measured by the present invention include tumor-associated antigens such as CA15-3, CA125, CA72-4, CA54/61, CA602, CA19-9, TPA, MCA, MSA, and ATM-1. Glycoproteins on the membrane of human milk fat grobule such as MAM-6 and GCDFP-15 may also be assayed by the same principle as the present invention.

CA (cancer antigen) 15-3 is a tumor-associated antigen recognized by such monoclonal antibodies as 115D8 discovered by Hilkens et al. which is an antibody against glycoprotein MAM-6 on human milk-fat globule membranes; and DF3 discovered by Kufe et al. which is an antibody prepared against a membrane-enriched fraction of a human breast carcinoma.

CA125 is a tumor-associated antigen discovered by Bast et al. which is recognized by monoclonal antibody OC125. OC125 is an antibody against cell line OVCA433 established from human ovarian serous cystadenocarcinoma.

CA72-4 is a tumor-associated antigen recognized by such monoclonal antibodies as B72.3 and CC49, both discovered by Colcher et al. B72.3 is a monoclonal antibody against membrane-enriched fraction of human breast carcinoma metastase to liver, and CC49 is a monoclonal antibody against TAG (tumor-associated glycoprotein) -72 purified from human colon carcinoma cell line LST174T.

CA54/61 is a tumor-associated antigen discovered by Nozawa et al., and is recognized by MA54 and MA61 which are monoclonal antibodies against human lung adenocarcinoma cell line C1509.

CA602 is a tumor-associated antigen discovered by Nozawa et al., and is recognized by F602-1 and F602-6 which are monoclonal antibodies against human ovarian mesonephroid cancer cell line RMG-II.

CA19-9 is a tumor-associated antigen discovered by Koprowski et al., and is recognized by 1116MS19-9 which is a monoclonal antibody against human colorectal carcinoma cell line SW1116.

TPA (tissue polypeptide antigen) is a single-chain polypeptide with molecular weight of about 22 kDa, extracted by Björklund et al., and is an oncofetal protein which is localized in cell membranes of tumor cell, placenta, and fetus.

MCA (mucinous carcinoma-associated antigen) is a tumor-associated antigen discovered by Stähli et al, and is recognized with monoclonal antibodies b-12, b-8 and b-15 against human breast cancer cell lines Hs0578T, SK-BR-3, MCF-7, and ZR-75-1.

MSA (mammary serum antigen) is a tumor-associated antigen discovered by Stacker et al., and is recognized by monoclonal antibody 3E1.2 against human breast carcinoma cells.

ATM-1 is a tumor-associated target antigen against human T-cell clone with activated killer activity 5B5, and is discovered by Kaieda et al. ATM-1 is also recognized by monoclonal antibody N1977 against human breast cancer cell line NBT-2.

The antibodies against such tumor-associated antigens may either be prepared by an appropriate conventional method or purchased from a suitable source.

The antibodies used in the method according to the present invention are not limited to purified antibodies, but include crude antibodies such as an antiserum and a salted out antibody containing an antibody having binding activity with said tumor-associated antigen. The antibody fragments which may be employed include the antibody moieties having binding activity with the target antigen such as Fab and F-(ab')$_2$. The antibody may either be polyclonal or monoclonal, and an appropriate antibody may be selected depending on the nature of the tumor-associated antigen to be assayed.

The antibody may either be physically or chemically bound to the solid phase. For example, the solid phase may be immersed and incubated in a solution containing the antibody at 4 to 56°C for 0.5 to 24 hours, and more preferably at 37 to 50°C for 1 to 2 hours to immobilize the antibody onto the solid phase. The concentration of the antibody employed may be in the range of from 1 microgram to 100 mg/ml and preferably from 1 to 100 micrograms/ml when the antibody is a purified antibody or a purified antibody fragment. The solid phase which has been immersed in the antibody solution may either be air- or freeze-dried. Insolubilization of the antibody onto the solid phase is not limited to the above-described manner, and all processes are included within the scope of the present invention wherein the antibody is immobilized onto the solid phase to substantially prevent the antibody from being separated from the solid phase in course of ordinary assay operations including application and rinsing operations.

The sheet-form solid phase having the antibody immobilized thereon may preferably by coated with a substance which does not participate in the antigen-antibody reaction between the antibody and the tumor-associated antigen in the nipple discharge in order to suppress non-specific reactions and more easily evaluate the result of the reaction. Such substances which do not participate in the antigen-antibody reaction may typically be a thermally denatured cytochrome c solution, BSA (bovine serum albumin), HSA (human serum albumin), animal serum, skim milk, and the like.

Amount of the antibody insolubilized onto the reaction area is determined in accordance with the concentration of the tumor-associated antigen in the nipple discharge to be assayed. For example, in a system wherein all the specimens containing the tumor-associated antigen above the predetermined concentration (cut off concentration) are to be determined positive, a convenient and accurate determination may be carried out by insolubilizing onto the solid phase such an amount of the antibody that all the antigen-binding sites of the antibody insolubilized within the reaction area would be substantially saturated or occupied by the tumor-associated antigen at the cut off concentration. In another example wherein the tumor-associated antigen contained in the specimen is to be quantitatively or semi-quantitatively assayed within a predetermined concentration range, a convenient and strict determination may be carried out by insolubilizing onto said reaction area such an amount of the antibody that all the antigen-binding sites of the antibody insolubilized onto said reaction area would be substantially saturated or occupied by the tumor-associated antigen contained in the specimen at the concentration at the upper limit of said range.

The labelling substance which may be employed in the method according to the present invention comprises the antibody or the fragment thereof against the tumor-associated antigen in the nipple discharge and an identifiable labelling agent bound to the antibody.

The identifiable labelling agents which may be bound to the antibody include pigment, radioisotope, enzyme and fluorescent material, among which the enzyme being preferred in view of stability, detection sensitivity, safety, and ease of treatment. The enzymes which may be employed include horseradish peroxidase, alkaline phosphatase, and the like, which are employed in conventional enzyme immunoassays.

When a plurality of tumor-associated antigens are simultaneously assayed by employing the assaying device according to the present invention which will be described below, it is preferable to use the same type of labelling agent for all the antigens assayed for the purpose of simple procedure.

The result of the reaction may be determined by assaying the amount or the activity of the labelling substance bound to the tumor-associated antigen on the solid phase after the immunoreaction. When an enzyme is used for the identifiable labelling agent, the assay of the labelling substance may be carried out by measuring the amount of the resulting reaction product of the labelled enzyme. The amount of the reaction product may be measured quantitatively by physical means using such measuring instruments as colorimeter, fluorophotometer, luminometer, and the like. The results may also be represented semi-quantitatively or qualitatively by subjecting the thus obtained measurements to a predetermined operational process.

When the reaction product is a chromophoric substance, the color developed may be either semi-quantitatively differentiated to several levels depending on its color strength, or qualitatively differentiated into positive and negative groups with the naked eye, the latter being more convenient.

The chromophoric substance whose color may be developed by the enzyme reaction may be any substance which are employed in conventional enzyme immunoassays. Illustrative chromophoric substances which may be used with peroxidase include 2,2'-adinodi(3-ethylbenzthiazoline)-6'-sulfonic acid (ABTS), o-phenylenediamine (OPD), tetramethylbenzidine (TMB), and 5-aminosalicylic acid. In the case of alkaline phosphatase, the enzyme may be reacted with such a substrate as p-nitrophenol phosphoric acid or phenyl phosphoric acid, and a phenol resulting from such an enzyme reaction may oxidatively conjugated with 4-aminoantipyrine to allow for color development.

For the purpose of storage after the determination of the results, the chromophoric substance may preferably be capable of depositing on the sheet-form solid phase and exhibiting a stable color tone which does not significantly vary with the lapse of time. Various chromophoric substances having such properties are known including diaminobenzidine which may be used with peroxidase, and 5-bromo-4-chloro-3-indoryl phosphonate (BCIP) which may be used with alkaline phosphatase.

In the method according to the present invention, the nipple discharge is usually collected with a suction pipette or a capillary, and the collected specimen is applied onto a predetermined reaction area of the solid phase. By using such procedure, the site of the disease can be estimated on the basis of the position of the mammary grand from which the particular specimen has been collected. In the case of collecting an extremely minute amount of nipple discharge, the specimen may be collected directly from the nipple onto the reaction area of the solid phase.

A substantially liquid-impermeable sheet-form solid phase is used as a carrier on which the antibody against the tumor-associated antigen is insolubilized, and the antigen-antibody reaction as well as the quantitative assay of the labelling substance are effected. The materials which may be used for such a solid phase include plastic resins such as polyester, polypropylene, vinyl chloride, polyethylene, polymethylpentene, etc., synthetic films such as Nylon film, nitrocellulose film, etc., and a paper coated with above-mentioned materials. Among those, plastic resins such as polyester, polypropylene and polyethylene, as well as Nylon film are particularly preferred since they rarely suffer from non-specific adsorption of components in the nipple discharge other than the tumor-associated antigen, and they may be readily handled during the assaying process including the rinsing. The liquid-impermeable sheet-form solid phase allows for an extremely minute amount of the specimen to be applied onto a relatively large area of the solid phase enabling the specimen to become in contact with a larger number of antibody molecules. At the same time, such a solid phase enables the specimen to be coated to a thinner layer allowing for a higher reaction efficiency. Effects of viscosity of the specimen on the assay results may also be minimized since the specimen applied onto the sheet-form solid phase exhibits little non-specific adsorption and may readily be removed by rinsing before or after the drying and solidifying of the reaction product on the solid phase regardless of the viscosity of the specimen. Consequently, effects on the results of the solidification of the reaction products or the nature of the specimen are minimized. By taking advantage of such properties of the liquid-impermeable solid phase, the solid phase having the specimen applied on the reaction area thereof may be stored until a satisfactory number of the specimens have been collected for further process. Such storage of the solid phase having the specimen applied thereon realizes an increased degree of freedom in such a situation as mass-screening wherein a large number of specimens are irregularly collected. The above-described effects may be enhanced by using a substantially liquid-impermeable plastic resin for the material of the sheet-form solid phase.

The specimen which may be used for the assay of the present invention is not essentially limited to the nipple discharge. Other biological liquids such as serum, plasma, urine, saliva, tear, perspiration, smear, and the like may also be employed for the assay according to the present invention, although the present method is most effective for a minute amount of specimen such as nipple discharge as apparent from the above description.

When the tumor-associated antigen in the nipple discharge is assayed in accordance with the present invention, nipple discharges from patients suffereing from early-stage breast cancer, wherein no tumor-associated antigen were detectable by conventional assay, often exhibited significantly high value. This indicates that the immunoassay according to the present invention may serve a potent auxililay means for diagnosing the breast cancer.

Positive rate of the early-stage breast cacer by using conventional assaying method has been quite low. For example, the positive rate for CA15-3 in blood of patients suffering from primary breast cancer at stage I and II was less than 10%, while it was 38 to 54% in blood of patients suffering from primary breast cacer at terminal stage IV or metastatic breast cancer, as disclosed in the afore-mentioned brochure titled "Centocore CA15-3 RIA kit" provided by TORAY-FUJI BIONICS, INC.

On the other hand, the nipple discharge assayed in accordance with the present invention with CA15-3 by using the cut-off value of 3000 U/ml exhibited positive rate of 100% (4 positive cases/4 positive cases assayed), specificity of 83% (5 negative cases/6 negative cases assayed), and rate of correct diagnosis of 90% (9 cases/10 cases assayed).

The assay of the tumor-associcated antigen in accordance with the present invention is quite simple and convenient since the entire process from the sample collection to the measurement may be carried out on the same solid phase, and the samples may be stored to enable the measurement at any desired occasion. High sentitivity as well as simple operation realized by the present invention have enabled the assay to be carried out at any occation when a patient with abnormal secretion of nipple discharge is discovered at mass screening or at the office or dispensary of a hospital. Such a speedy estimation of the cause for the abnormal nipple discharge may enable for a doctor to carry out an appropriate treatment at an early stage. As described above, the method according to the present invention has a clinical significance since a simple assay may be carried out without any special measuring apparatus to allow for the assay to be carried out at any facility.

Detection of the breast cancer at an early stage has been very difficult, and consequently, the cancer has frequently proceeded to far and gave rise to metastases to lymph nodes before it detection, inevitably requiring a large-scale operation such as mastectomy. In the case of the breast cancer which is detected at its early stage by the assay of tumor-associated antigen in the nipple discharge according to the present invention, the breast cancer may be treated by a relatively simple operation such as microdochectomy to allow for a relatively favorable recovery after the operation.

The device for assaying the tumor-associated antigen in the nipple discharge which is the second aspect of the present invention is described in detail with reference to the preferred embodiments illustrated in the drawings.

The sheet-form solid phase may have any practically convenient configuration and size so long as at least one reaction area having a predetermined size can be provided thereon. Four non-limiting preferable examples are set forth below.

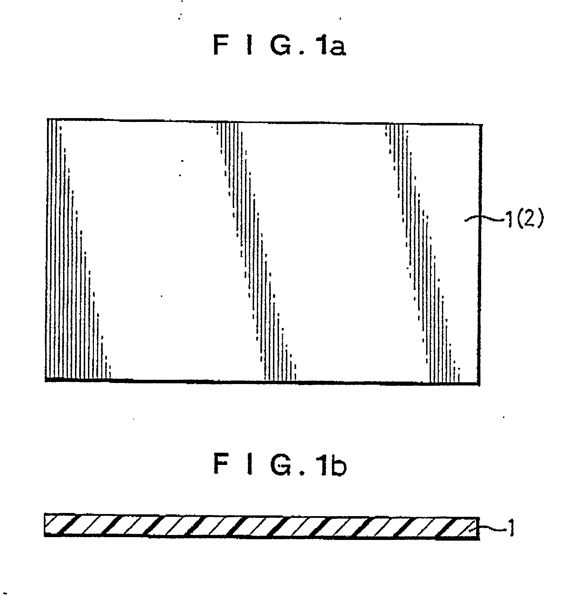

FIG. 1a is a plan view, and FIG. 1b is a cross-sectional view of a sheet 1 having one reaction area 2 thereon. The sheet 1 may preferably have a thickness of 0.1 to 2 mm, and any convenient size may be used to meet the handling requirements.

FIG. 2a is a plan view, and FIG. 2b is a cross-sectional view of the sheet 1 having a plurality of reaction areas 2. The reaction area 2 in this embodiment is a recess 5 provided in the sheet 1.

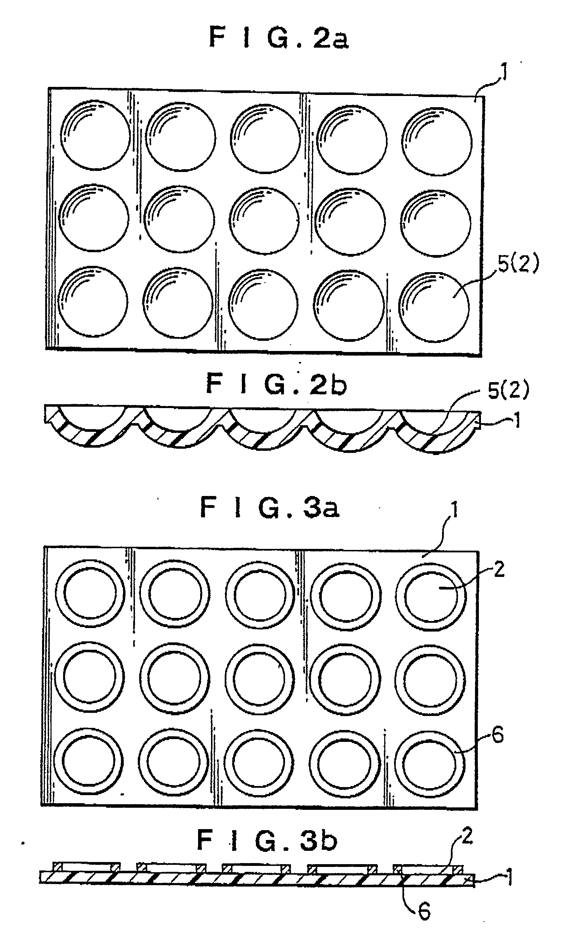

FIG. 3a is a plan view, and FIG. 3b is a cross-sectional view of the sheet 1 having a plurality of reaction areas 2. The reaction area 2 in this embodiment is surrounded by a ridge of frame 6.

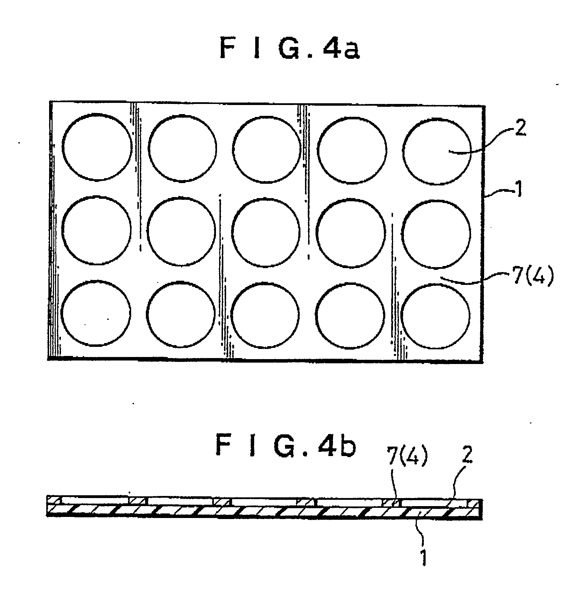

FIG. 4a is a plan view, and FIG. 4b is a cross-sectional view of the sheet 1 having a plurality of reaction areas 2. The reaction areas 2 in this embodiment are defined by a frame 7 covering the entire top surface of the sheet except for the reaction areas 2.

The size and the shape of the reaction area may be selected in accordance with the amount of the nipple discharge applicable thereon. For example, 5 microliters of the specimen may generally applied to an area of 0.01 to 3.0 $cm^2$. This area corresponds to a circle having a diameter of about 1 to 20 mm, although a diameter of 2 to 15 mm is preferable for a convenient operation and an easy determination. Of cause, these values may vary in accordance with the amount of the specimen used for the assay. The amount of the specimen required may also vary in accordance with the sensitivity of the assay and the range of the concentration to be assayed, and the size of the reaction area is accordingly varied. The non-limiting shape of the reaction area may preferably be a circle, ellipse, square, rectangle, and other quadrilateral for the purpose of a convenient operation including the application and rinsing as well as an easy determination.

The reaction area may be defined by merely insolubilizing the antibody onto a predetermined limited area of the solid phase, although it is more preferable that the predetermined reaction area of the solid phase is surrounded by a frame or a ridge provided on the solid phase or a recess provided in the sheet-form solid phase. The provision of such an area may readily be carried out when the solid phase is prepared from a substantially liquid-impermeable plastic resin.

When the reaction area is defined by insolubilizing the antibody against the tumor-associated antigen to be assayed onto a predetermined limited area of the solid phase, a plurality of tumor-associated antigen may be simultaneously assayed by insolubilizing different types of antibodies onto different reaction areas of one sheet-form solid phase.

The provision of the ridge or the frame surrounding the predetermined reaction area on the sheet-form solid phase may be carried out be press molding the ridge or by printing the frame with a water-repellent paint.

The reaction area may also be defined by covering the top surface of the solid phase except for the reaction area by means of a frame comprising an appropriate material such as aluminum foil 4 as shown in FIGS. 4a and 4b. More illustratively, such provision of the reaction area may be carried out by preparing an aluminum foil having an appropriate thickness in the range of 0.05 to 1.0 mm having openings whose size and shape are consistent with those of the reaction area, and heat bonding the aluminum foil onto the solid phase by using a hot-melt adhesive.

The reaction area may also be provided as a recess by such means as press molding as shown in FIGS. 2a and 2b.

It is to be noted that the reaction area of the predetermined size may also be provided by various means other than those described above.

The present invention is further illustrated by the following non-limiting examples.

## EXAMPLES

Example 1: Measurement of various tumor-associated antigens in nipple discharge by using antibody-immobilized polyester sheets - Comparison with conventional methods

Antibodies against various tumor-associated antigens were insolubilized onto polyester sheets, and the tumor-associated antigens in nipple discharge samples were measured by using the thus prepared sheets. The samples were also measured by conventional methods for the purpose of comparison.

(1) Preparation of the antibody-insolubilized sheet

An aluminum sheet having a plurality of circular openings with an inner diameter of 10 mm blanked at an interval of 15 mm was bonded to a polyester sheet (N500, manufactured by Somar K.K) by using a hot-melt adhesive. The thus prepared sheet was fully washed and dried. The antibodies listed in Table 1 were adjusted to concentrations of from 1 to 5 mg/ml with 76 mM phosphate buffer saline (hereinafter abbreviated as PBS), pH 6.4. The polyester sheet was immersed in the antibody solution, incubated at 45°C for 1 hour, rinsed in distilled water, thoroughly drained, further incubated at room temperature for 1 hour in PBS to which 10% BSA (bovine serum albumin) has been added, thoroughly drained, and air dried to obtain the antibody-insolubilized sheet.

(2) Preparation of the enzyme-labelled antibodies

The enzyme-labelled antibody employed for CA15-3 was prepared as described below. The enzyme-labelled antibodies for other tumor-associated antigens were also prepared in similar manners except that the antibodies listed in Table 1 were used for each of the tumor-associated antigens.

A 5 mg portion of horseradish peroxidase (grade 1-C, TOYOBO K.K., hereinafter referred to HRPO) was dissolved in 1 ml of 0.3 M aqueous solution of sodium hydrogencarbonate, and 0.1 ml of 1% ethanol solution of dinitrofluorobenzene was added to the solution and allowed to react at room temperature for 1 hour. 1 ml of 60 mM sodium periodate solution was then added to the solution and allowed to react for 30 minutes, and 1 ml of 0.16 M ethylene glycol solution was then added and allowed to react for 1 hour, and the thus obtained solution was dialyzed against 0.01M sodium carbonate buffer (hereinafter abbreviated as SCB), pH 9.5. To this solution, 5 mg of antibody against CA15-3, namely, DF3 was added and allowed to react at room temperature for 3 hours. 5 mg of sodium boronhydride was added to the solution and allowed to react overnight at 4°C, and the thus obtained solution was dialyzed against PBS to obtain an HRPO-labelled anti-CA15-3 antibody.

Table 1

| Antibodies Used for Measurement | | |
|---|---|---|
| Marker | Antibody labelled with Enzyme | Antibody insolubilized onto solid phase |
| CA15-3 | DF3 | 115D8 |
| CA125 | OC125 | OC125 |
| CA72-4 | B72.3 | CC49 |
| CA54/61 | MA54 | MA61 |
| CA602 | F602-6 | F602-1 |
| CA19-9 | 1116NS19-9 | 1116NS19-9 |
| TPA | TI62 | T162 |

(3) Preparation of standard solutions

(a) Preparation of standard solution for CA15-3

To human milk which has been ultracentrifuged at 40,000 x g to remove fats, PMSF and aprotinin were added to inhibit proteolysis, and the resulting solution was concentrated by ultrafiltrations The resulting concentrate was appropriately diluted and the concentration of CA15-3 was evaluated in accordance with the method designated in the kit shown in Table 4. This solution was diluted to various concentrations as shown in Table 3 with 10% BSA-added PBS to prepare standard solutions.

(b) Preparation of standard solutions for other tumor-associated antigens

The standard solutions for various tumor-associated antigens other than CA15-3 were prepared as described below by using culture media of the cancer cell lines shown in Table 2, respectively.

Table 2

| Cancer Cell Lines Employed for the Preparation of Standard Solutions | |
|---|---|
| Marker | Cancer cell line (origin) |
| CA125 | RMG-II, ovarian mesonephroid cancer cell line |
| CA72-4 | C1509, lung adenocarcinoma cell line |
| CA54/61 | C1509, lung adenocarcinoma cell line |
| CA602 | RMG-II, ovarian mesonephroid cancer cell line |
| CA19-9 | SW1116, colorectal carcinoma cell line |
| TPA | RMG-II, ovarian mesonephroid cancer cell line |

Cells in the culture medium of the cancer cell line were removed with filter paper (No. 131, manufactured by Advantech Toyo K.K.) to obtain culture supernatant. The culture supernatant was concentrated with ultrafilter (manufactured by Millipore Corporation), and purified by gel filtration column chromatography by using ACA22 (manufactured by Pharmacia-LKB). The purified solution was further concentrated by ultrafiltration, and the concentration of the tumor-associated antigen in the thus obtained concentrate was evaluated in accordance with the method designated in each kit of Table 4. The concentrate was diluted to various concentrations as shown in Table 3 with 10% BSA-added PBS to prepare standard solutions.

Table 3

| Concentration of Standard Solutions Employed for Measurements | |
|---|---|
| Marker | Concentration ,x $10^3$U/ml |
| CA15-3 | 0, 1, 2, 4, 8 |
| CA125 | 0, 2, 4, 8, 16 |
| CA72-4 | 0, 0.5, 1, 2, 4 |
| CA54/61 | 0, 1, 2, 4, 8 |
| CA602 | 0, 2, 4, 8, 16 |
| CA19-9 | 0, 0.75, 1.5, 3, 6 |
| TPA | 0, 1.5, 3, 6, 12 |

(4) Measurement of the tumor-associated antigens according to the present invention

Nipple discharge specimens were collected from 10 patients suffering from abnormal secretion of nipple discharge due to either breast cancer or benign diseases.

A 5 microliter portion of each specimen immediately after collection was respectively applied to cover the entire circular reaction area of the antibody-insolubilized sheet prepared in (1) and dried. 25 microliters of HRPO-labelled antibody which has been diluted by a factor of a hundred with 1% BSA-added PBS was added dropwise onto the dried sheet and allowed to react at room temperature for 30 minutes. The sheet was washed with distilled water to remove the reaction solution, and further washed by placing the sheet in a sealed container containing a cleaning solution (1 M saline to which 0.05% Tween 20 has been added) and violently agitating the cleaning solution. After thoroughly draining the sheet, 25 microliters of the substrate solution (0.5 mM TMB, 0.01% $H_2O_2$) was added onto the sheet and allowed to react for 10 minutes. The concentration of tumor-associated antigen in each specimen was semiquantitatively measured by referring to the colors developed using standard solutions as contrasts.

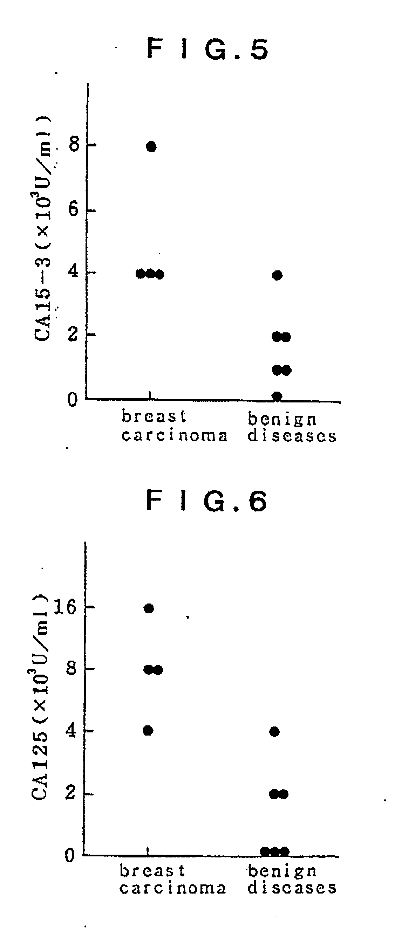

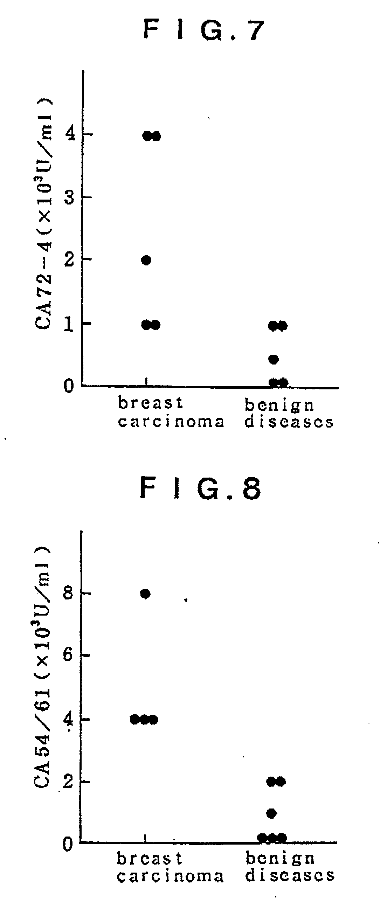

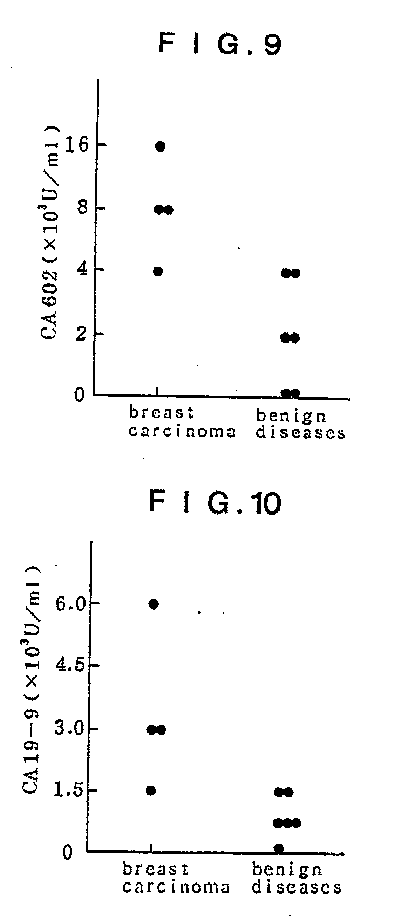

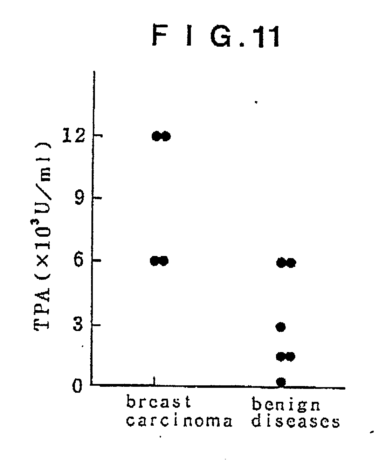

The results are shown in Table 5. The results are illustrated in FIGS. 5 to 12 separately for breast cancer and benign diseases.

(5) Measurement of the tumor-associated antigens according to conventional methods

The specimens measured in (4) were simultaneously measured by using the kits shown in Table 4 for each marker.

Table 4

| Conventional Methods | |
|---|---|
| Marker | Kit |
| CA15-3 | CA15-3 RI kit (Centocor Inc.) |
| CA125 | Immunoclone CA125 (TORAY-FUJI BIONICS INC.) |
| CA72-4 | CA72-4 RIA kit (Centocor Inc.) |
| CA54/61 | CA54/61 EIA kit (Mochida Pharmaceutical Co., Ltd.) |
| CA602 | CA602EIA kit (Mochida Pharmaceutical Co., Ltd.) |
| CA19-9 | CA19-9 RIA kit (Centocor Inc.) |
| TPA | TPA kit "Daiichi" II (Daiichi Radioisotope Research Institute) |

The results obtained are shown in Table 5 together with the measurements of the same specimens obtained in accordance with the present invention.

Table 5

| Comparison between the Measurements Obtained by the Present Invention and the Conventional Methods | | | | | | | | | | | |
|---|---|---|---|---|---|---|---|---|---|---|---|
| | | Measurements, x $10^3$ U/ml | | | | | | | | | |
| Marker | Method | 1 | 2 | 3 | 4 | 5 | 6 | 7 | 8 | 9 | 10* |
| CA15-3 | P.I.** | 4 | 4 | 4 | 8 | 2 | 1 | 4 | 1 | 2 | 0 |
| | C.M.*** | 6.8 | 4.0 | 5.3 | 6.4 | 1.7 | 1.5 | 6.1 | 1.2 | 2.8 | 0.5 |
| CA125 | P.I. | 16 | 8 | 4 | 8 | 4 | 2 | 0 | 2 | 0 | 0 |
| | C.M. | 18.3 | 9.3 | 5.2 | 8.3 | 4.9 | 2.6 | 1.4 | 2.0 | 0.3 | 0.5 |
| CA72-4 | P.I. | 4 | 4 | 2 | 1 | 1 | 0.5 | 1 | 0 | 1 | 0 |
| | C.M. | 4.3 | 5.7 | 2.8 | 0.8 | 1.1 | 0.9 | 1.3 | 0.2 | 1.9 | 0.1 |
| CA54/61 | P.I. | 4 | 8 | 4 | 4 | 2 | 1 | 2 | 0 | 0 | 0 |
| | C.M. | 4.9 | 8.8 | 5.3 | 5.4 | 2.3 | 1.7 | 1.8 | 0.6 | 0.4 | 0.6 |
| CA602 | P.I. | 16 | 4 | 8 | 8 | 4 | 2 | 0 | 2 | 4 | 0 |
| | C.M. | 15.0 | 5.2 | 9.6 | 8.7 | 4.9 | 1.7 | 0.8 | 2.8 | 3.4 | 1.7 |
| CA19-9 | P.I. | 3 | 3 | 6 | 1.5 | 0.75 | 0.75 | 1.5 | 0.75 | 1.5 | 0 |
| | C.M. | 4.4 | 5.1 | 7.3 | 1.4 | 0.6 | 0.8 | 1.7 | 0.8 | 1.3 | 0.1 |
| TPA | P.I. | 12 | 12 | 6 | 6 | 6 | 0 | 6 | 3 | 1.5 | 1.5 |
| | C.M. | 14.2 | 12.7 | 7.8 | 9.3 | 7.3 | 0.7 | 6.9 | 4.9 | 1.8 | 2.2 |

* specimen No., the specimens employed are different for each tumor marker.
** present invention, semi-quantitative
*** conventional method, quantitative.

As evident from Table 5, a good correlation was found between the measurements obtained by the present and conventional methods.

Moreover, the results shown in FIGS. 5 to 11 indicate that specimens exhibiting high values correspond to those of breast cancer, and specimens exhibiting low values correspond to those of benign diseases.

Example 2: Evaluation of effects on the results of the storage period after application of the specimens onto the polyester sheets

Specimens employed were those collected from patients suffering from abnormal nipple discharge, which were 3 specimens out of 10 specimens employed in Example 1.

A 5 microliter portion of each of the 3 specimens was applied immediately after its collection to cover the circular reaction areas of the antibody-insolubilized sheet prepared in Example 1(1), dried, and stored at 4°C. Concentration of the tumor-associated antigen was assayed for each of the specimens by means of the procedure described in Example 1(4).

The results are shown in Table 6.

Table 6

| Measurements after Storage | | | | | |
|---|---|---|---|---|---|
| | | Measurements, x $10^3$ U/ml Storage period, day | | | |
| Marker | Specimen | 0 | 1 | 7 | 14 |
| CA15-3 | A | 0 | 0 | 0 | 0 |
| | B | 2 | 2 | 2 | 2 |
| | C | 4 | 4 | 4 | 4 |
| CA125 | D | 0 | 0 | 0 | 0 |
| | E | 4 | 4 | 4 | 4 |
| | F | 16 | 16 | 16 | 16 |
| CA72-4 | G | 0 | 0 | 0 | 0 |
| | H | 1 | 1 | 2 | 1 |
| | I | 4 | | 4 | 4 |
| CA54/61 | J | 0 | 0 | 0 | 0 |
| | K | 2 | 2 | 2 | 2 |
| | L | 4 | 4 | 4 | 4 |
| CA602 | M | 0 | 0 | 0 | 0 |
| | N | 2 | 2 | 2 | 2 |
| | O | 8 | 8 | 8 | 8 |
| CA19-9 | P | 0 | 0 | 0 | 0 |
| | Q | 1.5 | 1.5 | 1.5 | 1.5 |
| | R | 6 | 6 | 6 | 6 |
| TPA | S | 0 | 0 | 0 | 0 |
| | T | 3 | 6 | 3 | 3 |
| | U | 12 | 12 | 12 | 12 |

The results shown in Table 6 indicate that no substantial effect on measurements is caused by the storage of the specimen-applied sheets.

Example 3: Comparison among various assaying procedures

(1) Preparation of the antibody-insolubilized sheets

Circular frames having a width of 1 mm and an inner diameter of 6 mm were silk screen-printed at an interval of 10 mm on a polyester sheet (N500, manufactured by Somar K.K.) with white water-repellent paint. After thoroughly rinsing with water and drying the sheet, the antibody was insolubilized onto the sheet by repeating the procedure of Example 1(1) to prepare the antibody-insolubilized sheet.

(2) Preparation of the enzyme-labelled antibodies

Bovine intestine alkaline phosphatase (ALP, manufactured by Berlinger Manheim) at a concentration of 10 mg/ml was mixed with the antibody to a concentration of 5 mg/ml. Clutaraldehyde was added to the mixture to a final concentration of 0.2% and allowed to react at $4°C$ for 2 hours. The resulting solution was dialyzed against 50 mM Tris-HCl buffer solution, pH 8.0 containing 0.1 M sodium chloride, 1 mM magnesium chloride, and 0.1% sodium azide at $4°C$ to obtain an ALP-labelled anti-CEA antibody (hereinafter referred to as ALP-labelled antibody).

(3) Measurement of the tumor-associated antigens

From patients suffering from abnormal nipple discharge, 3 specimens of the nipple discharge were collected and used for measurement.

(a) Procedure 1

A 5 microliter portion of each of three specimens was applied to cover the entire reaction area surrounded by the circular frame on the antibody-insolubilized sheet prepared in (1). After drying, 10 microliters of ALP-labelled antibody was added dropwise and allowed to react at room temperature for 30 minutes. The sheet was washed with distilled water to remove the reaction solution, and further washed by placing the sheet in a sealed container containing a cleaning solution (1 M saline to which 0.05% Tween 20 has been added) and violently agitating the cleaning solution. After thoroughly draining the sheet, 10 microliters of substrate solution (1 mg/ml 5-bromo-4-chloro-3-indoryl phosphate (BCIP), 1 mM magnesium chloride, 0.1 M boric acid-sodium carbonate buffer solution, pH 9.8) were added dropwise and left to stand at room temperature for 30 minutes until the solution was dried. The concentration of the tumor-associated antigen in the specimen was quantitated by measuring the reflectivity within the circular frame with a reflectivity meter (Color Measuring System SZ-80, manufactured by Nihon Denshoku Industries K.K.) using contrasts prepared by coating standard solutions prepared by repeating the procedure of Example 1 instead of the specimen.

The results are shown in Table 7.

(b) Procedure 2

10 microliters of ALP-labelled antibody were applied dropwise within the circular frames of the antibody-insolubilized sheet prepared in (1). 5 microliters of the specimens used in (a) were coated thereon, and allowed to react for 30 minutes at room temperature. The sheet was washed with distilled water to remove the reaction solution, and further washed with cleaning solution. The concentration of the tumor-associated antigen contained in the specimen was quantitated by repeating the procedure of (a).

(c) Procedure 3

5 microliters of each of the specimens used in (a) were mixed with 10 microliters of the ALP-labelled antibody. The mixture was coated within the circular frame of the antibody-insolubilized sheet prepared in (1), and allowed to react for 30 minutes at room temperature. The sheet was washed with distilled water to remove the reaction solution, and further washed with the cleaning solution. The concentration of the tumor-associated antigen in the specimen was quantitated by repeating the procedure of (a).

The results are shown in Table 7.

Table 7

| Comparison among Assaying Method | | | | |
|---|---|---|---|---|
| | | Measurements, x $10^3$ U/ml Assaying procedure | | |
| Marker | Specimen | 1 | 2 | 3 |
| CA15-3 | A | 0 | 0 | 0 |
| | B | 2.4 | 2.6 | 2.5 |
| | C | 4.0 | 4.2 | 4.2 |
| CA125 | D | 0 | 0 | 0 |
| | E | 3.2 | 3.3 | 3.2 |
| | F | 8.1 | 7.8 | 8.0 |
| CA72-4 | G | 0 | 0 | 0 |
| | H | 1.2 | 1.1 | 1.0 |
| | I | 4.5 | 4.4 | 4.4 |
| CA54/61 | J | 0 | 0 | 0 |
| | K | 1.3 | 1.0 | 1.0 |
| | L | 8.1 | 8.0 | 8.2 |
| CA602 | M | 0 | 0 | 0 |
| | N | 2.5 | 2.3 | 2.4 |
| | O | 7.7 | 7.9 | 7.8 |
| CA19-9 | P | 0 | 0 | 0 |
| | Q | 2.9 | 2.9 | 3.0 |
| | R | 6.2 | 6.3 | 6.3 |
| TPA | S | 0 | 0 | 0 |
| | T | 3.0 | 3.1 | 3.1 |
| | U | 12.2 | 12.1 | 12.0 |

As apparent from the data shown in Table 7, the measurements obtained by the three procedures were substantially consistent.

Example 4: Measurement of the tumor-associated antigens in nipple discharge by directly adsorbing the nipple discharge onto the antibody-insolubilized polypropylene sheets

(1) Preparation of the antibody-insolubilized sheets

The procedure of Example 1(1) was repeated except that the polypropylene sheet employed was PP (manufactured by Sun Platech K.K.).

(2) Measurement of the tumor-associated antigens according to the present invention

Samples were taken from 10 patients suffering from abnormal nipple discharge.

The antibody-insolubilized portion of the polypropylene sheet was directly contacted with the nipple to collect and apply the specimen onto the sheet. The sheet was incubated at 37°C for 1 hour, immersed in PBS to which 0.5% skim milk has been added, incubated at 4°C overnight, and blotted with filter paper to remove water. The polypropylene sheet was then immersed in the HRPO-labelled antibody solution prepared in Example 1(2) which has been diluted by a factor of 100 to 1000 by 1% BSA-added PBS, and

14

incubated at room temperature for 1 hour. After removing water from the sheet, the sheet was washed by placing the sheet in a sealed container containing a cleaning solution (1 M saline to which 0.05% Tween 20 has been added) and violently agitating the cleaning solution for 1 hour. The cleaning solution was replaced in every 15 minutes. The thus washed polypropylene sheet was thoroughly drained. A substrate solution (0.5 mM TMB containing 0.01% $H_2O_2$) was added dropwise onto the polypropylene sheet, and incubated for 15 minutes. The areas exhibiting significant color-development was designated positive ( + ) and those exhibiting no significant color development was designated negative (-).

The results are shown in Table 8.

(3) Measurement of the tumor-associated antigens according to conventional methods

The procedure of Example 1(5) was repeated for the specimens assayed in (2).
The results are shown in Table 8 together with the results obtained in (2).

Table 8

| Comparison between the Measurements Obtained by the Present Invention and the Conventional Methods | | | | | | | | | | | |
|---|---|---|---|---|---|---|---|---|---|---|---|
| | | Measurements, x $10^3$ U/ml | | | | | | | | | |
| Marker | Method | 1 | 2 | 3 | 4 | 5 | 6 | 7 | 8 | 9 | 10* |
| CA15-3 | P.I.** | + | + | + | - | - | - | - | - | - | - |
| | C.M.*** | 4.0 | 5.3 | 6.4 | 1.7 | 2.4 | 2.6 | 1.2 | 1.0 | 0.6 | 0.8 |
| CA125 | P.I. | + | + | + | + | + | - | - | - | - | - |
| | C.M. | 8.3 | 6.9 | 8.1 | 4.2 | 10.2 | 3.5 | 1.3. | 0.5 | 1.0 | 0.2 |
| CA72-4 | P.I. | + | - | + | - | - | - | - | - | - | - |
| | C.M. | 2.5 | 0.9 | 3.8 | 1.0 | 0.8 | 0.9 | 0.5 | 0.1 | 0.2 | 0.2 |
| CA54/61 | P.I. | + | - | + | - | - | - | - | - | - | - |
| | C.M. | 2.7 | 1.8 | 3.5 | 4.3 | 1.5 | 0.6 | 0.2 | 0.8 | 1.0 | 0.4 |
| CA602 | P.I. | + | + | + | + | + | - | - | - | - | - |
| | C.M. | 10.2 | 9.6 | 4.8 | 12.3 | 11.5 | 0.6 | 3.9 | 2.4 | 1.9 | 0.5 |
| CA19-9 | P.I. | + | - | - | - | + | - | - | - | - | - |
| | C.M. | 3.2 | 1.3 | 1.0 | 0.8 | 3.8 | 0.7 | 1.2 | 0.4 | 0.2 | 0.5 |
| | P.I. | + | + | + | + | + | + | + | - | - | - |
| | C.M. | 10.7 | 8.8 | 3.0 | 7.2 | 5.4 | 4.3 | 6.9 | 2.1 | 1.9 | 2.5 |

* specimen No., the specimens employed are different for each tumor marker.
** present invention, semi-qualitative
*** conventional method, quantitative.

The data shown in Table 8 indicate a good correlation between the measurements obtained by the direct-adsorbing method of the present invention and the conventional methods.

## Claims

1. A method for assaying concentration of a tumor-associated antigen selected from the group consisting of CA (cancer antigen) 15-3, CA (cancer antigen) 125, CA (carbohydrate antigen) 72-4, CA (carbohydrate antigen) 54/61, CA (carbohydrate antigen) 602, CA (carbohydrate antigen) 19-9, TPA (tissue polypeptide antigen), MCA (mucinous carcinoma-associated antigen), MSA (mammary serum antigen), and ATM-1 contained in a minute amount of nipple discharge wherein the concentration of said tumor-associated antigen in the nipple discharge is assayed by means of an antigen-antibody reaction using an antibody or a fragment thereof against said tissue-associated antigen, and said antigen-antibody reaction is effected on a sheet-form solid phase.

2. The method according to claim 1 wherein the antibody or the fragment thereof against the tumor-associated antigen selected from the group consisting of CA15-3, CA125, CA72-4, CA54/61, CA602, CA19-9, TPA, MCA, MSA, and ATM-1 is insolubilized on said sheet-form solid phase.

3. The method according to claim 1 or 2 wherein said concentration of the tumor-associated antigen selected from the group consisting of CA15-3, CA125, CA72-4, CA54/61, CA602, CA19-9, TPA, MCA, MSA, and ATM-1 in the nipple discharge is assayed by

(a) insolubilizing and immobilizing the antibody and/or the fragment thereof against the tumor-associated antigen onto said sheet-form solid phase,

(b) collecting a specimen of the nipple discharge, and applying said specimen onto a predetermined reaction area of said solid phase to contact the specimen with the antibody and/or the fragment thereof insolubilized within said area,

(c) applying a labelling substance onto the predetermined reaction area before or after the drying and solidification of said specimen on said solid phase so that the labelling substance becomes in contact with said specimen, said labelling substance comprising said antibody and/or the fragment thereof against the tumor-associated antigen having an identifiable labelling agent bound thereto,

(d) rinsing said solid phase to remove portion of the specimen and the labelling substance which failed to bind with the antibody and/or the fragment thereof insolubilized onto the solid phase, and

(e) assaying the concentration of said tumor-associated antigen in the nipple discharge on the bases of the amount of the labelling substance bound to said solid phase.

4. The method according to claim 1 or 2 wherein said concentration of the tumor-associated antigen selected from the group consisting of CA15-3, CA125, CA72-4, CA54/61, CA602, CA19-9, TPA, MCA, MSA, and ATM-1 in the nipple discharge is assayed by

(a) insolubilizing and immobilizing the antibody and/or the fragment thereof against the tumor-associated antigen onto said sheet-form solid phase,

(b) applying a labelling substance onto said sheet-form solid phase, said labelling substance comprising the antibody and/or the fragment thereof against the tumor-associated antigen having an identifiable labelling agent bound thereto,

(c) collecting a specimen of the nipple discharge, and applying said specimen onto a predetermined reaction area of said solid phase to simultaneously effect the reaction between the specimen and the insolubilized andtibody and the reaction between the specimen and the labelling substance,

(d) rinsing said solid phase to remove portion of the specimen and the labelling substance which failed to bind with the antibody and/or the fragment thereof insolubilized onto the solid phase, and

(e) assaying the concentration of said tumor-associated antigen in the nipple discharge on the bases of the amount of the labelling substance bound to said solid phase.

5. The method according to claim 1 or 2 wherein said concentration of the tumor-associated antigen selected from the group consisting of CA15-3, CA125, CA72-4, CA54/61, CA602, CA19-9, TPA, MCA, MSA, and ATM-1 in the nipple discharge is assayed by

(a) insolubilizing and immobilizing the antibody and/or the fragment thereof against the tumor-associated antigen onto said sheet-form solid phase,

(b) collecting a specimen of the nipple discharge,

(c) mixing the specimen with a labelling substance comprising said antibody and/or the fragment thereof against the tumor-associated antigen having an identifiable labelling agent bound thereto,

(d) applying said mixture onto a predetermined reaction area of said solid phase to contact the specimen with the antibody and/or the fragment thereof insolubilized within said area,

(e) rinsing said solid phase to remove portion of the specimen and the labelling substance which failed to bind with the antibody and/or the fragment thereof insolubilized onto the solid phase, and

(f) assaying the concentration of said tumor-associated antigen in the nipple discharge on the bases of the amount of the labelling substance bound to said solid phase.

6. The method according to any of the claims 1 to 3 wherein said nipple discharge specimen is collected by directly bringing said sheet-form solid phase into contact with the nipple.

7. The method according to any of the claims 1 to 5 wherein said nipple discharge specimen is collected by means of a collecting device.

8. The method according to any of the claims 1 to 7 wherein said sheet-form solid phase is a substantially liquid-impermeable plastic sheet.

9. The method according to claim 8 wherein said plastic is selected from the group consisting of polyester, polypropylene, and polyethylene.

10. The method according to any of the claims 2 to 10 wherein the sheet-form solid phase onto which the antibody and/or the fragment thereof against the tumor-associated antigen selected from the group consisting of CA15-3, CA125, CA72-4, CA54/61, CA602, CA19-9, TPA, MCA, MSA, and ATM-1 is insolubilized is coated with a substance which does not participate inthe antigen-antibody reaction between the tumor-associated antigen to be assayed and the antibody and/or the fragment thereof against said tumor-associated antigen.

11. The method according to any of the claims 3 to 10 wherein said identifiable labelling agent is an enzyme, and said measurement of the labelling substance is carried out by assaying an amount of a substance which increases or decreases as a result of an enzyme reaction of said enzyme.

12. The method according to any of the claims 1 to 11 wherein the concentration of said tumor-associated antigen is semiquantitatively or qualitatively determined by naked eye or physical means.

13. The method according to any of the claims 1 to 11 wherein the concentration of said tumor-associated antigen is quantitatively determined by physical means.

14. A device for assaying a tumor-associated antigen selected from the group consisting of CA15-3, CA125, CA72-4, CA54/61, CA602, CA19-9, TPA, MCA, MSA, and ATM-1 in a minute amount of nipple discharge comprising a sheet-form solid phase having at least one reaction area defined thereon for an antigen-antibody reaction between the tumor-associated antigen in the nipple discharge and an antibody or a fragment thereof against said tumor-associated antigen.

15. The device according to claim 14 wherein said antibody and/or a fragment thereof against said tumor-associated antigen selected from the group consisting of CA15-3, CA125, CA72-4, CA54/61, CA602, CA19-9, TPA, MCA, MSA, and ATM-1 is insolubilized onto said reaction area.

16. The device according to claim 15 wherein said sheet-form solid phase further comprises a labelling substance insolubilized onto said reaction area, said labelling substance comprising the antibody and/or the fragment thereof against said tumor-associated antigen and an identifiable labelling agent bound thereto.

17. The device according to any of the claims 14 to 16 wherein said sheet-form solid phase is a substantially liquid-impermeable plastic sheet.

18. The device according to claim 17 wherein said plastic is selected from the group consisting of polyester, polypropylene, and polyethylene.

19. The device according to any of the claims 14 to 18 wherein said reaction area on said sheet-form solid phase is surrounded by a ridge or a frame provided on said sheet-form solid phase.

20. The device according to any of the claims 14 to 18 wherein said reaction area on said sheet-form solid phase is a recess provided in said sheet-form solid phase.

21. The device according to any of the claims 16 to 20 wherein said identifiable labelling agent is selected from the group consisting of a pigment, radio isotope, enzyme, and fluorescent material.

22. The device according to any of the claims 15 to 21 wherein said reaction area is coated with a substance which does not participate in the antigen-antibody reaction between the tumor-associated antigen to be assayed and the antibody and/or the fragment thereof against said tumor-associated antigen.

# F I G . 1a

~1(2)

# F I G . 1b

~1

# F I G. 2a

1

5(2)

# F I G. 2b

5(2)

1

# F I G. 3a

1

2

6

# F I G. 3b

2

1

6

# F I G . 4a

2

1

7(4)

# F I G . 4b

7(4)   2

1

EP 0 394 510 A1

# F I G . 5

CA15-3 ($\times 10^3$U/ml)

breast carcinoma | benign diseases

# F I G . 6

CA125 ($\times 10^3$U/ml)

breast carcinoma | benign diseases

EP 0 394 510 A1

# F I G . 7

# F I G . 8

F I G . 9

F I G . 10

# F I G. 11

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | CANCER, vol. 60, no. 12, 1988, pages 3008-3013; H. INAJI et al.: "Carcinoembryonic antigen estimation in nipple discharge as an adjunctive tool in the diagnosis of early breast cancer" * Whole article * | 1-5 | G 01 N 33/574 G 01 N 33/545 |
| Y | EP-A-0 287 030 (ABBOTT LABS) * Pages 2-3,7 * | 1-5 | |
| A | BIOLOGICAL ABSTRACTS, vol. 86, no. 6, 1988, page AB-687, abstract no. 60531, Biological Abstracts Inc., Philadelphia, PA, US; E. YAYOI et al.: "New diagnostic method for the detection of non-palpable (T.) breast cancer", & J JPN SOC CANCER THER 23(1): 74-78. 1988 * Abstract * | 1 | |
| A | EP-A-0 232 179 (CETUS CORP.) * Page 1, line 1 - page 4, line 33 * | 1 | **TECHNICAL FIELDS SEARCHED (Int. Cl.5)** G 01 N |

The present search report has been drawn up for all claims

| Place of search | Date of completion of the search | Examiner |
|---|---|---|
| THE HAGUE | 21-12-1989 | HITCHEN C.E. |